(19) Europäisches Patentamt
European Patent Office
Office européen des brevets

(11) Veröffentlichungsnummer: **0 032 242**
**B1**

(12) # EUROPÄISCHE PATENTSCHRIFT

(45) Veröffentlichungstag der Patentschrift: **14.12.83**

(21) Anmeldenummer: **80108229.8**

(22) Anmeldetag: **29.12.80**

(51) Int. Cl.³: **C 07 D 265/22**, C 07 D 265/20, C 07 D 417/04, C 07 D 413/04, C 07 D 413/06, A 01 N 43/86 // C07C103/84

(54) **4H-3,1-Benzoxazinderivate, Verfahren zu ihrer Herstellung und ihre Verwendung zur Bekämpfung unerwünschten Pflanzenwuchses.**

(30) Priorität: **05.01.80 DE 3000309**

(43) Veröffentlichungstag der Anmeldung: **22.07.81 Patentblatt 81/29**

(45) Bekanntmachung des Hinweises auf die Patenterteilung: **14.12.83 Patentblatt 83/50**

(84) Benannte Vertragsstaaten: **AT BE CH DE FR GB IT LI NL SE**

(56) Entgegenhaltungen:
**EP - A - 0 017 931**
**FR - A - 2 121 341**
**GB - A - 1 051 259**
**GB - A - 1 153 994**
**US - A - 3 357 977**
**US - A - 3 914 121**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Varwig, Juergen, Dr., Am Goetzelberg 1, D-6900 Heidelberg (DE)**
Erfinder: **Wuerzer, Bruno, Dr., Ruedigerstrasse 13, D-6701 Otterstadt (DE)**

**EP 0 032 242 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 4H-3,1-Benzoxazinderivate, Verfahren zu ihrer Herstellung und ihre Verwendung zur Bekämpfung unerwünschten Pflanzenwuchses

Die vorliegende Erfindung betrifft substituierte 4H-3,1-Benzoxazinderivate, ein Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

4H-3,1-Benzoxazin-3-one sind als Zwischenprodukte für die Synthese von pharmakologisch wirksamen Verbindungen (DE-A-1 670 375, DE-A-2 556 590), als pharmakologisch wirksame Verbindungen (FR-A-2 121 341) oder als herbizide Wirkstoffe bekannt (BE-A-648 259, US-A-3 970 652, GB-A-1 051 259 oder US-A-3 914 121). Insbesondere 4H-3,1-Benzoxazin-4-one, die in 2-Stellung einen trifluormethylsubstituierten Phenylrest tragen, sind herbizid wirksam und zeichnen sich, im Vergleich zum unsubstituierten 2-Phenyl-4H-3,1-benzoxazin-4-on, durch eine höhere Aktivität und ein breiteres Wirkungsspektrum aus. Allerdings sind diese Verbindungen wenig wirksam gegen Amaranthaceen und unerwünschte Arten aus der Familie der Leguminosen.

Ausserdem ist bekannt, dass 3-Isopropyl-2,1,3-benzothiadiazin-4-on-2,2-dioxid (Bentazon) herbizid wirksam ist (DE-PS 1 542 836).

Es wurde nun gefunden, dass 4H-3,1-Benzoxazinderivate der Formel I

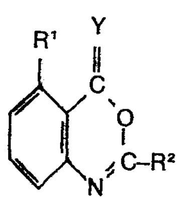

(I),

in der

Y Sauerstoff oder Schwefel und

$R^1$ Wasserstoff, wenn

$R^2$ für einen durch Halogenalkenyl, Halogenalkenyloxy oder Halogenalkenylmercapto mit jeweils bis zu 3 Kohlenstoffatomen einfach oder zweifach substituierten Phenylrest, wobei die Substituenten gleich oder verschieden sein können, oder für einen durch einen Halogenalkylrest mit bis zu 3 Kohlenstoffatomen, mit Ausnahme des Trifluormethylrestes, einfach substituierten Phenylrest steht, oder

$R^1$ Halogen, wenn

$R^2$ für einen durch Alkylmercapto, Alkylsulfonyl, Halogenalkylsulfinyl oder Halogenalkylsulfonyl mit jeweils bis zu 3 Kohlenstoffatomen einfach substituierten Phenylrest oder für einen einfach oder zweifach durch Halogenalkyl, Halogenalkenyl, Halogenalkenyloxy oder Halogenalkenylmercapto mit jeweils bis zu 3 Kohlenstoffatomen substituierten Phenylrest, wobei die Substituenten gleich oder verschieden sein können, steht, bedeuten, bei guter Verträglichkeit für eine Reihe von Kulturpflanzen herbizid wirksamer sind und ein breiteres Wirkungsspektrum aufweisen als bekannte 4H-3,1-Benzoxazinderivate.

In der Formel I bedeutet $R^1$ beispielsweise Wasserstoff, Fluor, Chlor, Brom oder Jod.

$R^2$ in Formel I bedeutet beispielsweise einen Phenylrest, der folgende Substituenten tragen kann:

Chlormethyl, Dichlormethyl, Trichlormethyl, Fluormethyl, Difluormethyl, Trifluormethyl, Chlorfluormethyl, Chlordifluormethyl, Dichlorfluormethyl, 1,1-Difluorethyl, Pentafluorethyl, 2,3,3-Trichlorallyl, 1,2-Dichlorvinyloxy, Trichlorvinyloxy, 1,2-Difluorvinyloxy, 2-Chlor-1,2-difluorvinyloxy, 1-Fluor-2,2-dichlorvinyloxy, 2,2-Dichlorvinyloxy, 2,2-Dibromvinyl, 2-Chlorvinyloxy, 2-Chlor-prop-1-enyloxy, 1,2-Dichlorvinylmercapto, Trichlorvinylmercapto, 1,2-Difluorvinylmercapto, 2-Chlor-1,2-difluorvinylmercapto, 2,2-Dichlorvinylmercapto oder 2-Chlorvinylmercapto, Methylmercapto, Ethylmercapto, $SO_2CH_3$, $SO_2C_2H_5$, $SO_2C_3H_7$, $SOCF_3$, $SOCF_2Cl$, $SO_2CF_3$, $SO_2CF_2Cl$.

Bevorzugte Verbindungen der Formel I sind solche, bei denen Y für Sauerstoff, $R^1$ für Wasserstoff und $R^2$ für einen einfach durch Halogenalkyl oder Halogenalkenyl mit jeweils bis zu 3 Kohlenstoffatomen, mit Ausnahme des Trifluormethylrestes, substituierten Phenylrest stehen, oder solche, bei denen Y für Sauerstoff, $R^1$ für Halogen und $R^2$ für einen einfach durch Halogenalkyl mit bis zu 3 Kohlenstoffatomen, insbesondere Trifluormethyl, Alkylmercapto, Alkylsulfonyl, Halogenalkylsulfinyl oder Halogenalkylsulfonyl mit jeweils bis zu 3 Kohlenstoffatomen substituierten Phenylrest stehen.

Man erhält die 4H-3,1-Benzoxazinderivate der Formel I durch Umsetzung einer gegebenenfalls substituierten Anthranilsäure der Formel II

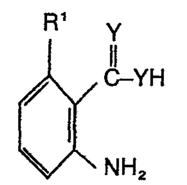

(II),

in der

$R^1$ und Y die oben genannten Bedeutungen haben, mit mindestens dem einfachen molaren Überschuss eines Carbonsäurehalogenids der Formel III

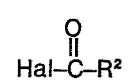

(III),

in der

$R^2$ die oben genannten Bedeutungen hat und Hal für Halogen, insbesondere Fluor, Chlor oder Brom steht, in einem aromatischen tertiären Amin als Lösungsmittel bei einer Temperatur im Bereich zwischen 10 und 60 °C.

Verwendet man Anthranilsäure und 3-(1,2-Dichlorvinyloxy)-benzoylchlorid als Ausgangsmaterialien, so kann der Reaktionsablauf durch folgendes Formelschema wiedergegeben werden:

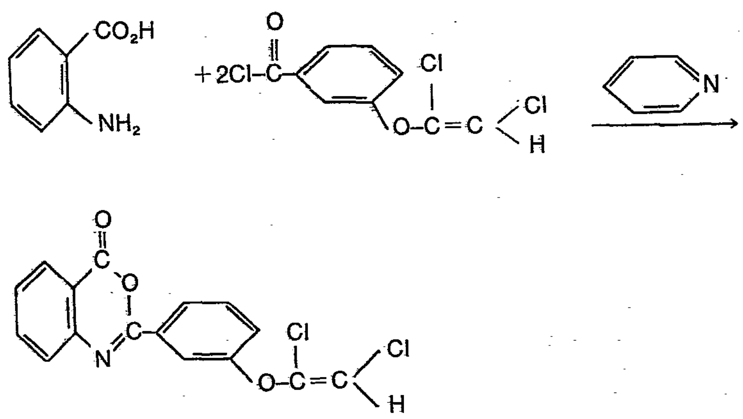

Zweckmässigerweise lässt man einen einfachen Überschuss des Carbonsäurehalogenids der Formel III in eine Lösung der Anthranilsäure der Formel II in der 5- bis 25fachen molaren Menge eines aromatischen Amins, bezogen auf Anthranilsäure, bei einer Temperatur zwischen 10 und 60 °C einlaufen und rührt dann 30 Minuten bei 25 °C nach (J. Chem. Soc. (C) 1968, 1593). Zur Aufarbeitung kann dann Eiswasser eingerührt und vom ausgefallenen Niederschlag abgesaugt werden. Ebenso kann das Carbonsäurehalogenid der Formel III vorgelegt werden.

Geeignete aromatische tertiäre Amine sind beispielsweise Pyridin, α-, β-, γ-Picolin, Lutidin, Chinolin und Acridin.

Die Benzoxazinderivate der Formel I können auch dadurch erhalten werden, dass man eine gegebenenfalls substituierte Anthranilsäure der Formel II

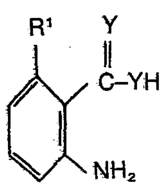

(II),

in der

$R^1$ und Y die oben angegebenen Bedeutungen haben, oder ein Alkalimetall- oder Erdalkalimetallsalz dieser Anthranilsäure mit ungefähr stöchiometrischen Mengen eines Carbonsäurehalogenids der Formel III

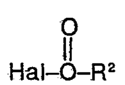

(III),

in der

$R^2$ die in Anspruch 1 genannten Bedeutungen hat und Hal für Halogen steht, in einem inerten organischen Lösungsmittel oder in Wasser und gegebenenfalls in Anwesenheit eines Säureakzeptors bei einer Temperatur im Bereich von 0 bis 60 °C zu einem Carbonamid der Formel IV

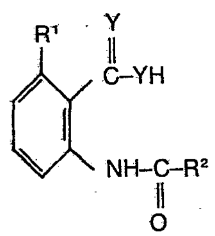

(IV),

in der

$R^1$, $R^2$ und Y die oben genannten Bedeutungen haben, umsetzt und dieses dann in Gegenwart eines wasserentziehenden Mittels bei einer Temperatur zwischen 30 und 150 °C cyclisiert.

Geeignete inerte Lösungsmittel für diese Umsetzung sind Kohlenwasserstoffe, wie Ligroin, Benzin, Toluol, Pentan, Hexan, Cyclohexan, Petrolether, Halogenkohlenwasserstoffe, wie Methylenchlorid, Chloroform, Tetrachlorkohlenstoff, 1,1- und 1,2-Dichlorethan, 1,1,1- und 1,1,2-Trichlorethan, Chlorbenzol, o-, m-, p-Dichlorbenzol, o-, m-, p-Chlortoluol, Nitrokohlenwasserstoffe, wie Nitrobenzol, Nitroethan, o-, m-, p-Chlornitrobenzol, Nitrile, wie Acetonitril, Butyronitril, Isobutyronitril, Ether, wie Diethylether, Di-n-propylether, Tetrahydrofuran, Dioxan, Ester wie Acetessigester, Ethylacetat oder Isobutylacetat, oder Amide, wie Formamid, Methylformamid oder Dimethylformamid.

Als Säureakzeptoren können alle üblichen Säurebindemittel verwendet werden. Hierzu gehören vorzugsweise Alkalihydroxide, Alkalicarbonate und tertiäre organische Basen. Besonders geeignet sind beispielsweise Natriumhydroxid, Natriumcarbonat, Natriumhydrogencarbonat, Triethylamin, Pyridin, Trimethylamin, α-, β-, γ-Picolin, Lutidin, N,N-Dimethylanilin, N,N-Dimethylcyclohexylamin, Chinolin, Tri-n-propylamin und Tri-n-butylamin. Das Säurebindemittel wird zweckmässigerweise in äquivalenten Mengen, bezogen auf Carbonsäurehalogenid der Formel III, eingesetzt.

Als wasserentziehende Mittel können symmetrische und gemischte Carbonsäureanhydride, wie Essigsäureanhydrid, Propionsäureanhydrid, Buttersäureanhydrid, Ameisensäureessigsäureanhydrid, Ameisensäurepropionsäureanhydrid, oder Essigsäurepropionsäureanhydrid, ferner Dicyclohexylcarbodiimide oder Thionylchlorid verwendet werden. Die Cyclisierung wird unter Zusatz der 1- bis 10fachen molaren Menge an wasserentziehendem Mittel, bezogen auf Carbonamid der Formel IV, durchgeführt.

Die Ausgangsstoffe der Formeln II und III werden in ungefähr stöchiometrischem Verhältnis eingesetzt, d.h. zweckmässigerweise in einem Unter- bzw. Überschuss von bis zu 10% Ausgangsstoff der Formel III gegenüber Ausgangsstoff der Formel II.

Zweckmässigerweise wird das Verfahren so durchgeführt, dass man über zwei Zuführungen das Carbonsäurehalogenid der Formel III und die äquivalente Menge an Säureakzeptor bei einer Temperatur zwischen 0 und 60 °C zu einer ungefähr äquivalenten Menge der Anthranilsäure der Formel II bzw. ihres Salzes in einem inerten organischen Lösungsmittel bzw. in Wasser zulaufen lässt. Dann rührt man 15 Minuten bei Raumtemperatur nach. Das Reaktionsgemisch wird dann gegebenenfalls eingeengt, in der Wärme mit 5n Salzsäure angesäuert, abgekühlt und abgesaugt (J. Org. Chem. 2, 396 (1944)), wobei eine N-Acyl-2-aminobenzoesäure erhalten wird. Diese kann dann in Gegenwart der 5- bis 10fachen molaren Menge an Carbonsäureanhydrid als wasserentziehendem Mittel, beispielsweise Acetanhydrid, durch Rühren – gegebenenfalls unter Abdestillation der entstandenen Essigsäure – bei einer Temperatur zwischen 30 und 150 °C zu dem gewünschten 4H-3,1-Benzoxazin cyclisiert werden. Zur Aufarbeitung entfernt man hierbei überschüssiges wasserentziehendes Mittel unter vermindertem Druck und kristallisiert gegebenenfalls zur Reinigung um. Anstelle der Anthranilsäure kann auch das Carbonsäurehalogenid vorgelegt werden. Verwendet man Dicyclohexylcarbodiimid oder Thionylchlorid als wasserentziehende Mittel, lässt sich die Cyclisierung mit der 1- bis 4fachen molaren Menge durchführen.

4H-3,1-Benzoxazine der Formel I, bei denen $R^2$ für einen durch Halogenalkenyloxy oder Halogenalkenylmercapto substituierten Phenylrest steht, werden zweckmässigerweise durch Umsetzung der Halogenalkenyloxy- bzw. Halogenalkenylmercapto-substituierten Benzoesäure in die entsprechenden Säurechloride (Houben-Weyl, Methoden der organischen Chemie, Bd. 8, S. 463 ff, 4. Auflage, Georg Thieme-Verlag, Stuttgart 1952), die dann mit gegebenenfalls substituierten Anthranilsäuren der Formel I auf bekannte Weise in die entsprechenden Amide umgewandelt werden, hergestellt. Letztere können dann durch Ringschluss in Gegenwart wasserabspaltender Agentien in substituierte 2-Phenyl-3,1-benzoxazin-4-one überführt werden.

Zur Isolierung der 4H-3,1-Benzoxazinderivate der Formel I aus der Reaktionsmischung kann man diese mit Wasser, verdünntem Alkali oder verdünnter Säure zur Abtrennung von Nebenprodukten, wie nichtumgesetzter Anthranilsäure, Säurechlorid bzw. Basenhydrochlorid, behandeln, trocknen und dann einengen. Gegebenenfalls können die Endprodukte auch durch Umkristallisation oder Chromatographie gereinigt werden.

Die Herstellung der Carbonsäurehalogenide der Formel III lässt sich am Beispiel des 3-(1',2'-Dichlorvinyloxy)-benzoylchlorids erläutern:

a) 185 Teile Trichlorethylen werden in eine Suspension von 180 Teilen 3-Hydroxybenzoesäuremethylester-natriumsalz in 200 Teilen Ethylenglykolmonoethylether in einem Autoklav eingeführt und 2 Stunden bei 150 °C gerührt. Nach dem Abziehen des Lösungsmittels unter vermindertem Druck wird der viskose Rückstand in 300 Teilen Methylenchlorid aufgenommen, mit Aktivkohle filtriert und über neutrales Aluminiumoxid chromatographiert. Nach der Entfernung des Methylenchlorids unter vermindertem Druck wird der Rückstand über eine Vigreux-Kolonne bei 125–131 °C/0,6 mbar destilliert, wobei der 3-(1',2'-Dichlorvinyloxy)-benzoesäuremethylester vom $n^{25}_D$ = 1,5419 erhalten wird.

b) 30 Teile des oben erhaltenen Esters werden in einer Lösung von 7,2 Teilen Kaliumhydroxid in 100 Teilen Wasser und 9 Teilen Ethanol 2 Stunden unter Rückfluss gerührt. Anschliessend wird das Reaktionsgemisch einmal mit 50 Teilen Ether extrahiert, in 70 Volumenteile eiskalte, 12%ige Salzsäure eingerührt, abgesaugt und mit Wasser gewaschen. Nach dem Trocknen werden 24 Teile 3-(1',2'-Dichlorvinyloxy)-benzoesäure vom Fp. 137–141 °C erhalten.

c) 81,6 Teile 3-(1',2'-Dichlorvinyloxy)-benzoesäure in 620 Teilen 1,2-Dichlorethan werden mit 48 Teilen Thionylchlorid und einem Tropfen Pyridin versetzt und 3 Stunden unter Rückfluss gerührt. Nach dem Einengen unter vermindertem Druck werden 85 Teile 3-(1',2'-Dichlorvinyloxy)-benzoylchlorid vom Fp. 50–54 °C isoliert.

Die folgenden Beispiele erläutern die Herstellung der erfindungsgemässen 4H-3,1-Benzoxazinderivate der Formel I. Gewichtsteile verhalten sich zu Volumenteilen wie Kilogramm zu Liter.

Beispiel 1

Herstellung von 2-(3'-Trifluormethylphenyl)-5-chlor-4H-3,1-benzoxazin-4-on

a) 23 Teile 3-Trifluormethylbenzoylfluorid und 12,2 Teile Triethylamin werden über 2 Zuführungen gleichzeitig bei 20 bis 25 °C zu einer Lösung von 20,6 Teilen 6-Chloranthranilsäure in 310 Teilen 1,2-Dichlorethan eingeführt und 10 Minuten nachgerührt. Dann wird das Reaktionsgemisch einmal mit 70 Volumenteilen 1n Salzsäure extrahiert, wobei ein farbloser Niederschlag ausfällt. Dieser wird in Essigester gelöst, die organische Phase wird nochmals mit 1n Salzsäure und mit Wasser gewaschen, über Magnesiumsulfat getrocknet und eingeengt; hierbei werden 32 Teile N-3'-Trifluormethylbenzoyl-6-chloranthranilsäure vom Fp. 217–219 °C erhalten. In gleicher Weise wird mit der zuvor erhaltenen 1,2-Dichlorethanphase verfahren, wobei nochmals 7 Teile des gleichen Produkts mit einem Fp. von 210–218 °C erhalten werden.

b) 25 Teile N-3'-Trifluormethylbenzoyl-6-chloranthranilsäure werden in 250 Teilen Essigsäureanhydrid zweieinhalb Stunden unter Rückfluss gerührt. Nach dem Einengen unter vermindertem Druck wird der Rückstand in Methylenchlorid aufgenommen, zweimal mit je 100 Volumenteilen 0,5n Natronlauge und mit Wasser gewaschen und getrocknet. Anschliessend wird die Lösung über neutrales Aluminiumoxid chromatographiert, wobei 23 Teile 2-(3'-Trifluormethylphenyl)-5-chlor-4H-3,1-benzoxazin-4-on vom Fp. 116–120 °C erhalten werden. Ausbeute: 97% d.Th.

Beispiel 2

Herstellung von 2-(3'-Trifluormethylphenyl)-5-fluor-4H-3,1-benzoxazin-4-on

a) 23 Teile 3-Trifluormethylbenzoylfluorid und 15,3 g Dimethylcyclohexylamin werden gleichzeitig über zwei Zuführungen bei 25 bis 30 °C in eine Lösung von 18,6 Teilen 6-Fluoranthranilsäure in 350 Teilen Essigester eingeführt. Das Reaktionsgemisch wird 30 Minuten nachgerührt, einmal mit 1n Salzsäure und mit Wasser extrahiert. Nach dem Trocknen und Einengen werden 38 Teile N-3'-Trifluormethylbenzoyl-6-fluoranthranilsäure vom Fp. 183–185 °C erhalten; Ausbeute: 97% d.Th.

b) 25 Teile Thionylchlorid werden bei Raumtemperatur zu einer Suspension von 23 Teilen N-3'-Trifluormethylbenzoyl-6-fluoranthranilsäure in 150 Teilen 1,2-Dichlorethan gegeben und dann eine Stunde unter Rückfluss gerührt. Anschliessend wird die Reaktionsmischung eingeengt, in Methylenchlorid aufgenommen, einmal mit Wasser und zweimal mit je 100 Volumenteilen 0,5n Natronlauge extrahiert. Nach dem Trocknen, Einengen, Absaugen und Waschen mit wenig Cyclohexan werden 20 Teile 2-(3'-Trifluormethylphenyl)-5-fluor-4H-3,1-benzoxazin-4-on vom Fp. 115–121 °C isoliert; Ausbeute: 92% d.Th.

Nach entsprechenden Verfahren können folgende 4H-3,1-Benzoxazinderivate der Formel I hergestellt werden:

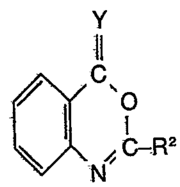

| Nr. | $R^2$ | Y | Fp. [°C] |
|---|---|---|---|
| 3 | 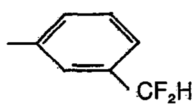 | O | |
| 4 | 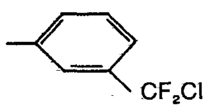 | O | 106–108 |
| 5 | 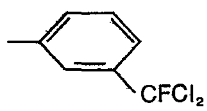 | O | 92– 95 |
| 6 | 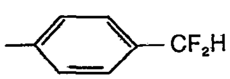 | S | |
| 7 | 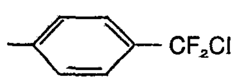 | O | |
| 8 | 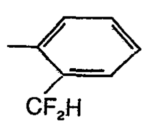 | O | |
| 9 | 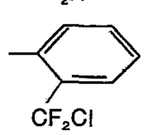 | S | |

| Nr. | $R^2$ | Y | Fp. [°C] |
|---|---|---|---|
| 10 | 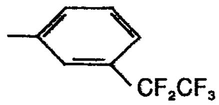 | O | |
| 11 | 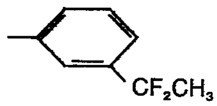 | O | |
| 12 | 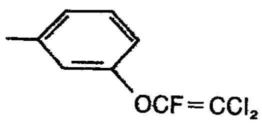 | O | |
| 13 | 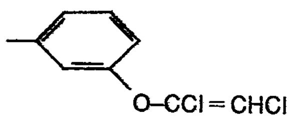 | O | 109–112 |
| 14 | 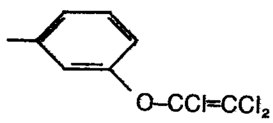 | O | |
| 15 | 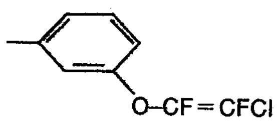 | S | |
| 16 | 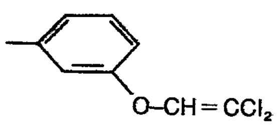 | O | |
| 17 | 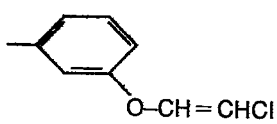 | O | |
| 18 | 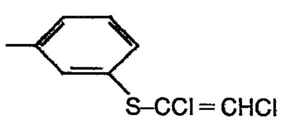 | O | |
| 19 | 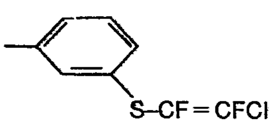 | O | |
| 20 | 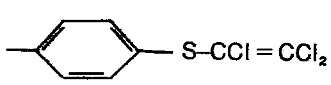 | O | |

| Nr. | $R^1$ | $R^2$ | Y | Fp. [°C] |
|---|---|---|---|---|
| 21 | F | 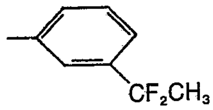 | O | |
| 22 | Br | 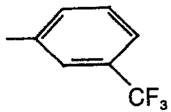 | O | |
| 23 | J | 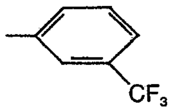 | O | |
| 24 | Cl | 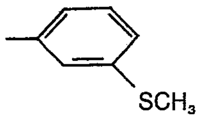 | O | 144-147 |
| 25 | Cl | 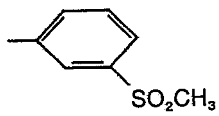 | O | 222–228 |
| 26 | Cl | 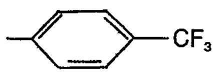 | O | 148–150 |
| 27 | Cl | 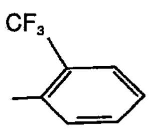 | O | 106–108 |
| 28 | F | 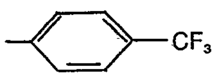 | O | 136–139 |
| 29 | Cl | 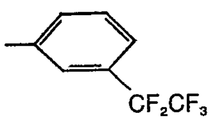 | O | |
| 30 | Cl | 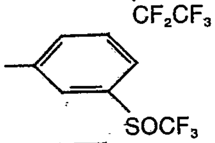 | O | |
| 31 | Cl | 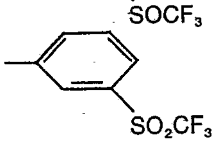 | O | |
| 32 | Cl | 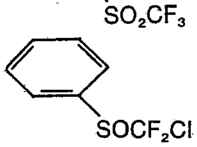 | O | |
| 33 | Cl | 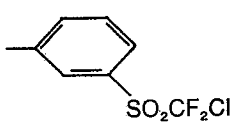 | O | |
| 34 | Cl | 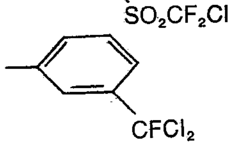 | | 138–140 |
| 35 | Cl | 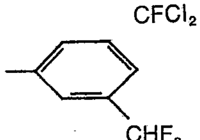 | O | 117–119 |
| 36 | Cl | 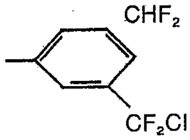 | O | 96–100 |

| Nr. | R¹ | R² | Y | Fp. [°C] |
|---|---|---|---|---|
| 37 | Br | 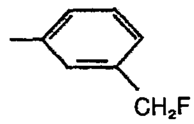 | S | |
| 38 | Cl | 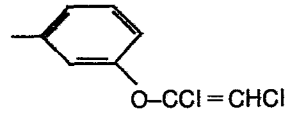 | O | 119–123 |
| 39 | F | 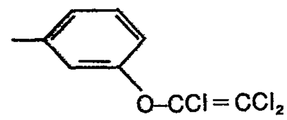 | O | |
| 40 | Cl | 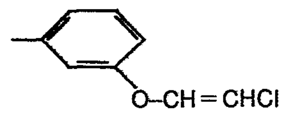 | O | |
| 41 | Cl | 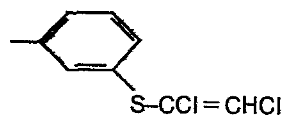 | O | |
| 42 | F | 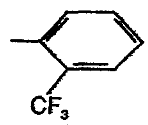 | O | 94– 98 |
| 43 | F | 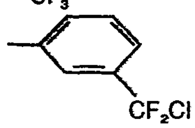 | O | 129–133 |
| 44 | Cl | 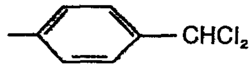 | O | 180–183 |

Die erfindungsgemässen Wirkstoffe können beispielsweise in Form von direkt versprühbaren Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel wie z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten zwischen 0,1 und 95 Gewichtsprozent, vorzugsweise 0,5 und 90 Gewichtsprozent, Wirkstoff.

Beispiele für die Formulierungen sind:

I. Man vermischt 90 Gewichtsteile der Verbindung gemäss Beispiel 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung gemäss Beispiel 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Rizinusöl besteht. Durch Ausgiessen und feines Verteilen der Lösung in 100 000 Gewichtsteilen Wasser erhält man eine wässrige Dispersion, die 0,02 Gewichtsprozent des Wirkstoffs enthält.

III. 20 Gewichtsteile der Verbindung Nr. 13 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Rizinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Die Applikation der Wirkstoffe bzw. der Mittel kann im Vorauflaufverfahren oder bei Nachauflaufanwendung erfolgen. Dabei können die Mittel auf den Standort gebracht werden, bevor die unerwünschten Pflanzen aus den Samen gekeimt oder aus vegetativen Pflanzenteilen ausgetrieben haben, oder sie werden auf die Blätter der unerwünschten Pflanzen und der Kulturpflanzen appliziert. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,1 bis 15 kg/ha und mehr, wobei sich die höheren Dosen besonders zur totalen Bekämpfung von Vegetationen eignen.

Der Einfluss verschiedener Vertreter der erfindungsgemässen 4H-3,1-Benzoxazinderivate auf das Wachstum von unerwünschten Pflanzen im Vergleich zu bekannten Wirkstoffen wird durch Gewächshausversuche gezeigt:

Als Kulturgefässe dienten Plastikblumentöpfe mit 300 $cm^3$ Inhalt und lehmigem Sand mit etwa 1,5% Humus als Substrat. Die Samen der Testpflanzen entsprechend Tabelle 1 wurden nach Arten getrennt flach eingesät oder es wurden vorgekeimte Jungpflanzen oder Stecklinge bzw. angekeimte Knollen von Cyperus esculentus eingesetzt.

Generell wurden die Pflanzen zum Zwecke der Nachauflaufbehandlung bis zu einer Höhe von 3 bis 10 cm gezogen und danach behandelt. Die Wirkstoffe wurden auf die Sprossteile der Pflanzen und die nicht völlig von Pflanzen abgedeckte Bodenfläche, in Wasser als Verteilungsmittel suspendiert oder emulgiert, mittels fein verteilender Düsen gespritzt. Die Versuchstöpfe wurden dann in verschiedenen Temperaturbereichen der Gewächshausanlagen aufgestellt, wobei für wärmeliebende Arten 20 bis 30 °C und für solche gemässigter Klimate 10 bis 20 °C bevorzugt wurden. Die Versuchsperiode erstreckte sich über 2 bis 4 Wochen. Während dieser Zeit wurden die Pflanzen gepflegt, und ihre Reaktion auf die einzelnen Wirkstoffe wurde ausgewertet. Die Auswertung der Versuche wurde nach einer Skala von 0 bis 100 vorgenommen. Dabei bedeutet 0 keine Schädigung und 100 völliges Absterben zumindest der oberirdischen Sprossteile.

Als Vergleichsmittel wurden die bekannten Wirkstoffe

2-(3'-Trifluormethyl-phenyl)-4H-3,1-benzoxazin-4-on

($V_1$; US-A-3 914 121; US-A-3 970 652) und 3-Isopropyl-2,1,3-benzothiadiazin-4-on-2,2-dioxid ($V_2$; DE-PS 1 542 836) getestet.

Tabelle 1 – Liste der Pflanzennamen

| Botanischer Name | Abkürzung in Tabelle | Deutscher Name |
|---|---|---|
| Amaranthus retroflexus | Amar. retrofl. | Zurückgekrümmter Fuchsschwanz |
| Desmodium tortuosum | Desmod. tort. | |
| Euphorbia geniculata | Euph. genic. | südamerik. Wolfsmilchart |
| Matricaria spp. | Matric. spp. | Kamillearten |
| Mercurialis annua | Mercur. annua | einjähriges Bingelkraut |
| Triticum aestivum | Tritic. aest. | Weizen |
| Zea mays | – | Mais |
| Solanum nigrum | | schwarzer Nachtschatten |
| Chenopodium album | | weisser Gänsefuss |

Tabelle 2 – Selektive herbizide Wirkung von 4H-3,1-Benzoxazinderivaten in Cerealien bei Nachauflaufanwendung im Gewächshaus

| Wirkstoff Nr. | Aufwandmenge (kg a.S./ha) | Testpflanzen und Schädigung (%) Tritic. aest. | Zea mays | Amar. retrofl. | Desmod. tort. | Euph. genic. | Matric. spp. | Mercur. annua |
|---|---|---|---|---|---|---|---|---|
| 1 | 0,25 | 0 | 0 | – | 80 | 100 | 100 | 100 |
| | 0,5 | 0 | 0 | 85 | 100 | 100 | 100 | 100 |
| 2 | 0,25 | 0 | 2 | – | 68 | 99 | 100 | 100 |
| | 0,5 | 0 | 8 | 50 | 100 | 100 | 100 | 100 |
| $V_1$ | 0,25 | 0 | 0 | – | 8 | 2 | 65 | 68 |
| | 0,5 | 0 | 0 | 11 | 8 | 19 | 88 | 80 |
| $V_2$ | 0,25 | 0 | 0 | – | 0 | 0 | 100 | 5 |
| | 0,5 | 0 | 0 | 22 | 0 | 0 | 100 | 5 |

0 = keine Schädigung
100 = Pflanzen abgestorben

Tabelle 3 – Selektive herbizide Wirksamkeit von 4H-3,1-Benzoxazinderivaten bei Nachauflaufanwendung im Gewächshaus

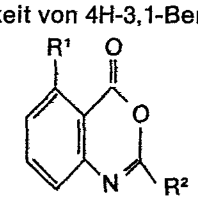

| Wirkstoff Nr. | $R^1$ | $R^2$ | Aufwandmenge (kg a.S./ha) | Testpflanzen und Schädigung (%) Triticum aestivum | Chenopodium album |
|---|---|---|---|---|---|
| 13 | H | 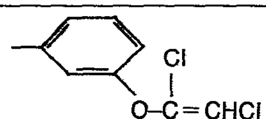 | 0,5 | 0 | 100 |
| 34 | Cl | 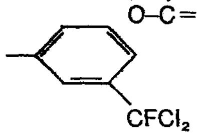 | 1,0 | 0 | 100 |
| 4 | H | 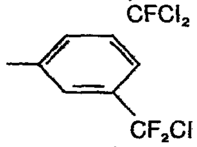 | 0,5 | 0 | 98 |
| 36 | Cl | 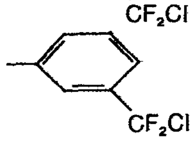 | 0,5 | 0 | 98 |
| 43 | F | 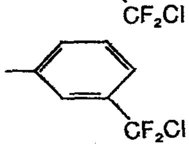 | 0,5 | 0 | 90 |

Tabelle 4 – 4H-3,1-Benzoxazinderivate zur selektiven Bekämpfung von breitblättrigen Unkräutern in Mais und Getreidekulturen bei Nachauflaufanwendung im Gewächshaus

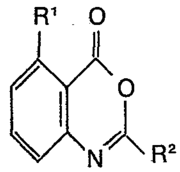

| Nr. | $R^1$ | $R^2$ | Aufwandmenge (kg a.S./ha) | Testpflanzen und Schädigung (%) Triticum aestivum | Zea mays | Desmodium tortuosum | Solanum nigrum |
|---|---|---|---|---|---|---|---|
| 28 | F | 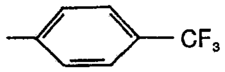 | 0,5 | 0 | 5 | 100 | 100 |
| 26 | Cl | 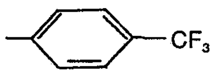 | 0,5 | 0 | 0 | 100 | 100 |
| 5 | H | 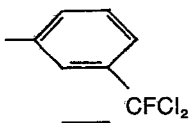 | 0,5 | 0 | – | – | 100 |
| 36 | Cl | 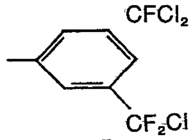 | 0,5 | 0 | 0 | 100 | – |
| 43 | F | 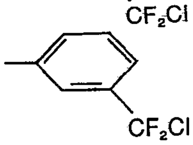 | 0,5 | 0 | 0 | 100 | – |

In Anbetracht der guten Verträglichkeit der Wirkstoffe und der Vielseitigkeit der Applikationsmethoden können die erfindungsgemässen Verbindungen oder diese enthaltende Mittel ausser bei den in Tabelle 1 aufgeführten Nutzpflanzen noch in einer weiteren grossen Zahl von Kulturpflanzen zur Beseitigung unerwünschten Pflanzenwuchses eingesetzt werden.

In Betracht kommen beispielsweise die folgenden Kulturen:

| Botanischer Name | Deutscher Name |
|---|---|
| Allium cepa | Küchenzwiebel |
| Ananas comosus | Ananas |
| Arachis hypogaea | Erdnuss |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weisse Rüben |
| Brassica rapa var. silvestris | Rübsen |
| Camellia sinensis | Teestrauch |
| Carthamus tinctorius | Saflor – Färberdistel |
| Carya illinoinensis | Pekannussbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |

| Botanischer Name | Deutscher Name |
|---|---|
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süsskartoffeln |
| Juglans regia | Walnussbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- und Rohrkolbenhirse |
| Petroselinim crispum spp. tuberosum | Wurzelpetersilie |
| Picea abies | Rotfichte |
| Abies alba | Weisstanne |
| Pinus spp. | Kiefer |
| Pisum sativum | Gartenerbse |
| Prunus avium | Süsskirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preiselbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die 4H-3,1-Benzoxazinderivate der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan 1,3-dionderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon
5-Methylamino-4-chlor-2-(3-trifluormethylphenyl)-3(2H)-pyridazinon
5-Methylamino-4-chlor-2-(3-α,α,β,β-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3-trifluormethylphenyl)-3(2H)-pyridazinon
5-Methoxy-4-chlor-2-(3-trifluormethylphenyl)-3(2H)pyridazinon
5-Amino-4-brom-2-(3-methylphenyl)-3(2H)-pyridazinon
4,5-Dimethoxy-2-(3-α,α,β-trifluor-β-bromethoxyphenyl)-3-(2H)-pyridazinon
3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze
1-Methoxymethyl-3-(1-methylethyl)-2,1,3-benzothiadiazin-4(3H)-on-2,2-dioxid
1-Methoxymethyl-8-chlor-3-(1-methylethyl-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Cyan-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-pyridino-[3,2-e]2,1,3-thiadiazin-(4)-on-2,2-dioxid
N-(1-Ethylpropyl)-2,6-dinitro-3,4-dimethylanilin
N-(1-Methylethyl)-N-ethyl-2,6-dinitro-4-trifluormethyl-anilin
N-n-Propyl-N-β-chlorethyl-2,6-dinitro-4-trifluormethyl-anilin
N-n-Propyl-N-cyclopropylmethyl-2,6-dinitro-4-trifluormethyl-anilin
N-Bis-(n-propyl)-2,6-dinitro-3-amino-4-trifluormethylanilin
N-Bis-(n-propyl)-2,6-dinitro-4-methyl-anilin
N-Bis-(n-propyl)-2,6-dinitro-4-methylsulfonyl-anilin
N-Bis-(n-propyl)-2,6-dinitro-4-methylsulfonylanilin
Bis-(β-chlorethyl)-2,6-dinitro-4-methyl-anilin
N-Ethyl-N-(2-methylallyl)-2,6-dinitro-4-trifluormethyl-anilin
N-Methylcarbaminsäure-3,4-dichlorbenzylester
N-Methylcarbaminsäure-2,6-di-tert.-butyl-4-methylphenyl-ester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionanilid
Ethyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-N'-3-chlor-4-fluorphenylcarbamoyloxy)-phenyl)-carbamat
Ethyl-N-(3-N'-3,4-difluorphenylcarbamoyloxy)-phenyl)-carbamat
Methyl-N-(3-(N'-3,4-difluorphenylcarbamoyloxy)-phenyl)-carbamat
N-3-(4-Fluorphenoxycarbonylamino)-phenylcarbaminsäure-methylester
N-3-(2-Methylphenoxycarbonylamino)-phenylcarbaminsäure-ethylester
N-3-(4-Fluorphenoxycarbonylamino)-phenyl-thiocarbaminsäuremethylester
N-3-(2,4,5-Trimethylphenoxycarbonylamino)-phenyl-thiocarbaminsäure-methylester
N-3-(Phenoxycarbonylamino)-phenyl-thiocarbaminsäure-methylester
N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester

N,N-Di-sec.-butyl-thiolcarbaminsäure-ethylester
N,N-Di-sec.-butyl-thiolcarbaminsäure-benzylester
N-Ethyl-N-cyclohexyl-thiolcarbaminsäure-ethylester
N-Ethyl-N-bicyclo[2.2.1]heptyl-thiolcarbaminsäureethylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-(3-methyl-hexahydro-1-H-azepin-1)-carbothiolat
S-Benzyl-(2,3-dimethylhexahydro-1-H-azepin-1)-carbothiolat
S-Ethyl-(3-methylhexahydro-1-H-azepin-1)-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz
α-Methyl-α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure
2,3,5-Triiodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Di-natrium-3,6-endoxohexahydro-phthalat
4-Amino-3,5,6-trichlor-picolinsäure (Salze)
2-Cyan-3-(N-methyl-N-phenyl)-amino-acrylsäure-ethylester
2-[4-(4′-Chlorphenoxy)-phenoxy]-propionsäure-isobutylester
2-[4-(2′,4′-Dichlorphenoxy)-phenoxy]-propionsäuremethylester
2-[4-(4′-Trifluormethylphenoxy)-phenoxy]-propionsäuremethylester
2-[4-(2′-Chlor-4′-trifluorphenoxy)-phenoxy]-propionsäure-Natriumsalz
2-[4-(3′,5′-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäureisopropylester
4-(4′-Trifluormethyl-phenoxy)-penten-2-carbonsäureethylester
2-Chlor-4-ethylamino-6-isopropylamino-1,3,5-triazin
2-Chlor-4-ethylamino-6-(amino-2′-propionitril)-1,3,5-triazin
2-Chlor-4-ethylamino-6,2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin
2-Azido-4-methylamino-6-isopropylamino-1,3,5-triazin
2-Methylthio-4-ethylamino-6-isopropylamino-1,3,5-triazin
2-Methylthio-4-ethylamino-6-tert.-butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.-butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.-butyl-3-methylthio-4,5-dihydro-1,2,4-triazin-5-on
1-Methyl-3-cyclohexyl-6-dimethylamino-1,3,5-triazin-2,4-dion
3-tert.-Butyl-5-chlor-6-methyluracil
3-tert.-Butyl-5-brom-6-methyluracil
3-Isopropyl-5-brom-6-methyluracil
3-sec.-Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil
2-Methyl-4-(3′-trifluormethylphenyl)-tetrahydro-1,2,4-oxadiazin-3,5-dion
2-Methyl-4-(4′-fluorphenyl)-tetrahydro-1,2,4-oxadiazin-3,5-dion
3-Amino-1,2,4-triazol
1-Allyloxy-1-(4-bromphenyl)-2-[1′,2′,4′-triazolyl-(1′)]-ethan (Salze)
1-(4-Chlorphenoxy)-3,3-dimethyl-1-(1H-1,2,4-triazol-1-yl)-butan-2-on
N,N-Diallylchloracetamid
N-Isopropyl-2-chloracetanilid
N-(Butin-1-yl-3)-2-chloracetanilid
2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid

2-Methyl-6-ethyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-isobutoxymethyl-2-chloracetanilid
2,6-Diethyl-N-methoxymethyl-2-chloracetanilid
2,6-Diethyl-N-(n-butoxymethyl)-2-chloracetanilid
2,6-Diethyl-N-ethoxycarbonylmethyl-2-chloracetanilid
2,3,6-Trimethyl-N-(pyrazol-1-yl-methyl)-2-chloracetanilid
2,3-Dimethyl-N-isopropyl-2-chloracetanilid
2-(2-Methyl-4-chlorphenoxy)-N-methoxy-acetamid
2-(-Naphthoxy)-N,N-diethylpropionamid
2,2-Diphenyl-N,N-dimethylacetamid
N-Benzyl-N-isopropyl-trimethylacetamid
α-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyl-trifluormethansulfonanilid
5-Acetamido-4-methyl-trifluormethansulfonanilid
2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)
Pentachlorphenol-Natriumsalz
2,5-Dichlorphenyl-4′-nitrophenylether
2,4,6-Trichlorphenyl-4′-nitrophenylether
2-Fluor-4,6-dichlorphenyl-4′-nitrophenylether
2-Chlor-4-trifluormethylphenyl-4′-nitrophenylether
2,4′-Dinitro-4-trifluormethyl-diphenylether
2,4-Dichlorphenyl-3′-methoxy-4′-nitro-phenylether
2-Chlor-4-trifluormethylphenyl-3′-ethoxy-4′-nitro-phenylether
2-Chlor-4-trifluormethylphenyl-3′-carboxy-4′-nitro-phenylether (Salze)
2-Chlor-4-trifluormethylphenyl-3′-ethoxycarbonyl-4′-nitro-phenylether
2-Chlor-4-trifluormethylphenyl-3′-(2-fluorethoxy)-4′-nitro-phenylether
2-Chlor-4-trifluormethylphenyl-3′-ethoxycarbonyl-methylthio-4′-nitrophenylether
2-Chlor-4-trifluormethyl-3′-methoxycarbonyl-4′-nitrophenylether
2,4,6-Trichlorphenyl-3-ethoxycarbonyl-methylthio-4′-nitro-phenylether
2,4-Dichlorphenyl-3′-methoxycarbonyl-4′-nitrophenylether
2,4-Dichlorphenyl-3′-carboxy-4′-nitrophenylether
2-(3,4-Dichlorphenyl)-4-methyl-1,2,4-oxadiazolidin-3,5-dion
2-(3-tert.-Butylcarbamoyloxy-phenyl)-4-methyl-1,2,4-oxadiazolidin-3,5-dion
2-(3-i-Propylcarbamoyloxy-phenyl)-4-methyl-1,2,4-oxazolidin-3,5-dion
2-Phenyl-3,1-benzoxazinon-(4)
(4-Bromphenyl)-3,4,5,9,10-pentaazatetracyclo-[5,4,1,0$^{2,6}$,0$^{8,11}$]-dodeca 3,9-dien

2-Ethoxy-2,3-dihydro-3,3-dimethyl-5-benzofuranyl-methan-sulfonat
2-Ethoxy-2,3-dihydro-3,3-dimethyl-5-benzofuranyl-dimethyl-aminosulfonat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)
2-sec.-Butyl-4,6-dinitrophenol (Salze, Ester)
2-sec.-Butyl-4,6-dinitrophenol-acetat
2-tert.-Butyl-4,6-dinitrophenol-acetat
2-tert.-Butyl-4,6-dinitrophenol (Salze)
2-tert.-Butyl-5-methyl-4,6-dinitrophenol (Salze
2-tert.-Butyl-5-methyl-4,6-dinitrophenol-acetat
2-sec.-Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethylharnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-α,α,β,β-Tetrafluorethoxyphenyl)-3,3-dimethylharnstoff
1-(3-tert.-Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethylharnstoff
1-[4-(4′-Chlorphenoxy)-phenyl]-3,3-dimethyl-harnstoff

1-[4-(4′-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.-Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
1-(4-Benzyloxyphenyl)-3-methyl-3-methoxy-harnstoff
Imidazolidin-2-on-1-carbonsäure-isobutylamid
1,2-Dimethyl-3,5-diphenylpyrazolium-methylsulfat
1,2-4-Trimethyl-3,5-diphenylpyrazoliummethylsulfat
1,2-Dimethyl-4-brom-3,5-diphenylpyrazolium-methylsulfat
1,3-Dimethyl-4-(3,4-dichlorbenzoyl)-5-[(4-methylphenyl)-sulfonyloxy]-pyrazol
1-Acetyl-3-anilino-4-methoxycarbonyl-5-methylpyrazol
3-Anilino-4-methoxycarbonyl-5-methyl-pyrazol
3-tert.-Butylamino-4-methoxycarbonyl-5-methyl-pyrazol
2,3,5-Trichlor-pyridinol-(4)
1-Methyl-3-phenyl-5-(3′-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1′-Dimethyl-4,4′-dipyridylium-di-methylsulfat
1,1′-Di-(3,5-dimethylmorpholin-carbonylmethyl)-4,4′-dipyridylium-dichlorid
1,1′-Ethylen-2,2′-dipyridylium-dibromid

3-[1(N-Ethoxyamino)-propyliden]-6-ethyl-3,4-dihydro-2H-pyran-2,4-dion
3-[1-(N-Allyloxyamino)-propyliden]-6-ethyl-3,4-dihydro-2H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)

2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

α-Naphthoxyessigsäuremethylester
2-[4-(5′-Brompyridyl-2-oxy)-phenoxy]-propionsäureethylester
2-[4-(5′-Iodpyridyl-2-oxy)-phenoxy]-propionsäureethylester
2-[4-(5′-Iodpyridyl-2-oxy)-phenoxy]-propionsäuren-butylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
2-[4-(4′-Chlorphenoxymethyl)-phenoxy]-propionsäuremethylester
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
2-[1-(N-ethoxamino)-butyliden]-5-(2-ethylthiopropyl)-3-hydroxy-cyclohexen-(2)-on-(1) (Salze)
2-[1-(N-ethoxamino)-butyliden]-5-(2-phenylthiopropyl)-3-hydroxy-cyclohexen-(2)-on-(1) (Salze)

Gibellerinsäure (Salze)
Dinatrium-methylarsonat
Mononatriumsalz der Methylarsonsäure
N-Phsophon-methyl-glycin (Salze)
N,N-Bis-(phosphonmethyl)-glycin (Salze)
2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
O,O-Diisopropyl-5-(2-benzosulfonylamino-ethyl)-phosphordithioat
2,3-Dihydro-5,6-dimethyl-1,4-dithiin-1,1,4,4-tetraoxid
5-tert.-Butyl-3-(2,4-dichlor-5-isopropoxyphenyl)-1,3,4-oxadiazolon-(2)

4,5-Dichlor-2-trifluormethyl-benzimidazol (Salze)
1,2,3,6-Tetrahydropyridazin-3,6-dion (Salze)
Bernsteinsäure-mono-N-dimethylhydrazin (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanyl-ethylketon
2-[1-(2,5-Dimethylphenyl)-ethylsulfonyl]-pyridin-N-oxid
Natriumchlorat
Ammoniumrhodanid
Calciumcyanamid

Ausserdem ist es nützlich, die erfindungsgemässen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nichtphytotoxische Öle und Ölkonzentrate zugesetzt werden.

**Patentansprüche für die Vertragsstaaten:** BE, CH, DE, FR, GB, IT, LI, NL, SE

1. 4H-3,1-Benzoxazinderivate der Formel I

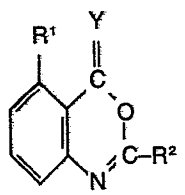

(I),

in der

Y Sauerstoff oder Schwefel und

$R^1$ Wasserstoff, wenn

$R^2$ für einen durch Halogenalkenyl, Halogenalkenyloxy oder Halogenalkenylmercapto mit jeweils bis zu 3 Kohlenstoffatomen einfach oder zweifach substituierten Phenylrest, wobei die Substituenten gleich oder verschieden sein können, oder für einen durch einen Halogenalkylrest mit bis zu 3 Kohlenstoffatomen, mit Ausnahme des Trifluormethylrestes, einfach substituierten Phenylrest steht, oder

$R^1$ Halogen, wenn

$R^2$ für einen durch Alkylmercapto, Alkylsulfonyl, Halogenalkylsulfinyl oder Halogenalkylsulfonyl mit jeweils bis zu 3 Kohlenstoffatomen einfach substituierten Phenylrest oder für einen einfach oder zweifach durch Halogenalkyl, Halogenalkenyl, Halogenalkenyloxy oder Halogenalkenylmercapto mit jeweils bis zu 3 Kohlenstoffatomen substituierten Phenylrest, wobei die Substituenten gleich oder verschieden sein können, steht, bedeuten.

2. 4H-3,1-Benzoxazinderivate der Formel I gemäss Anspruch 1, dadurch gekennzeichnet, dass $R^1$ für Wasserstoff, Y für Sauerstoff und $R^2$ für einen einfach durch Halogenalkyl oder Halogenalkenyl mit jeweils bis zu 3 Kohlenstoffatomen, mit Ausnahme des Trifluormethylrestes, substituierten Phenylrest stehen.

3. 4H-3,1-Benzoxazinderivate der Formel I gemäss Anspruch 1, dadurch gekennzeichnet, dass $R^1$ für Halogen, Y für Sauerstoff und $R^2$ für einen durch Halogenalkyl, Alkylmercapto, Alkylsulfonyl, Halogenalkylsulfinyl oder Halogenalkylsulfonyl mit jeweils bis zu 3 Kohlenstoffatomen einfach substituierten Phenylrest stehen.

4. Herbizid, enthaltend inerte Zusatzstoffe und ein 4H-3,1-Benzoxazinderivat der Formel I gemäss Anspruch 1.

5. Verfahren zur Bekämpfung unerwünschten Pflanzenwuchses, dadurch gekennzeichnet, dass man ein Herbizid, enthaltend mindestens ein 4H-3,1-Benzoxazinderivat der Formel I gemäss Anspruch 1, verwendet.

6. Verfahren zur Herstellung von 4H-3,1-Benzoxazinderivaten der Formel I gemäss Anspruch 1, dadurch gekennzeichnet, dass man eine gegebenenfalls substituierte Anthranilsäure der Formel II

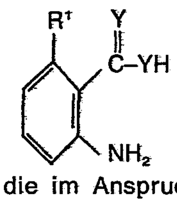

(II),

in der $R^1$ und Y die im Anspruch 1 genannten Bedeutungen haben, mit mindestens dem einfachen molaren Überschuss eines Carbonsäurehalogenids der Formel III

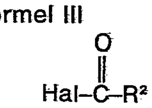

(III),

in der $R^2$ die im Anspruch 1 genannten Bedeutungen hat und Hal für Halogen steht, in einem aromatischen tertiären Amin als Lösungsmittel bei einer Temperatur im Bereich zwischen 10 und 60 °C umsetzt.

7. Verfahren zur Herstellung von 4H-3,1-Benzoxazinderivaten der Formel I gemäss Anspruch 1, dadurch gekennzeichnet, dass man eine gegebenenfalls substituierte Anthranilsäure der Formel II

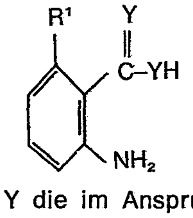

(II),

in der $R^1$ und Y die im Anspruch 1 genannten Bedeutungen haben, oder ein Alkalimetall- oder Erdalkalimetallsalz dieser Anthranilsäure mit stöchiometrischen Mengen eines Carbonsäurehalogenids der Formel III

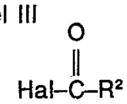

(III),

in der $R^2$ die im Anspruch 1 genannten Bedeutungen hat und Hal für Halogen steht, in einem inerten organischen Lösungsmittel oder in Wasser und gegebenenfalls in Anwesenheit eines Säureakzeptors bei einer Temperatur im Bereich von 0 bis 60 °C zu einem Carbonamid der Formel IV

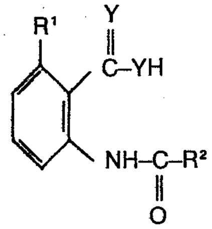

(IV),

in der $R^1$, $R^2$ und Y die im Anspruch 1 genannten Bedeutungen haben, umsetzt und dieses dann in Gegenwart eines wasserentziehenden Mittels bei einer Temperatur im Bereich zwischen 30 und 150 °C cyclisiert.

**Patentansprüche für den Vertragsstaat:** AT

1. Herbizid, enthaltend ein 4H-3,1-Benzoxazinderivat der Formel I

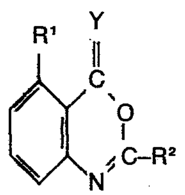

(I),

in der

Y Sauerstoff oder Schwefel und

$R^1$ Wasserstoff, wenn

$R^2$ für einen durch Halogenalkenyl, Halogenalkenyloxy oder Halogenalkenylmercapto mit jeweils bis zu 3 Kohlenstoffatomen einfach oder zweifach substituierten Phenylrest, wobei die Substituenten gleich oder verschieden sein können, oder für einen durch einen Halogenalkylrest mit bis zu 3 Kohlenstoffatomen, mit Ausnahme des Trifluormethylrestes, einfach substituierten Phenylrest steht, oder

$R^1$ Halogen, wenn

$R^2$ für einen durch Alkylmercapto, Alkylsulfonyl, Halogenalkylsulfinyl oder Halogenalkylsulfonyl mit jeweils bis zu 3 Kohlenstoffatomen einfach substituierten Phenylrest oder für einen einfach oder zweifach durch Halogenalkyl, Halogenalkenyl, Halogenalkenyloxy oder Halogenalkenylmercapto mit jeweils bis zu 3 Kohlenstoffatomen substituierten Phenylrest, wobei die Substituenten gleich oder verschieden sein können, steht, bedeuten, als Wirkstoff.

2. Herbizid, enthaltend ein 4H-3,1-Benzoxazinderivat der Formel I gemäss Anspruch 1, dadurch gekennzeichnet, dass $R^1$ für Wasserstoff, Y für Sauerstoff und $R^2$ für einen einfach durch Halogenalkyl oder Halogenalkenyl mit jeweils bis zu 3 Kohlenstoffatomen, mit Ausnahme des Trifluormethylrestes, substituierten Phenylrest stehen.

3. Herbizid, enthaltend ein 4H-3,1-Benzoxazinderivat der Formel I gemäss Anspruch 1, dadurch gekennzeichnet, dass $R^1$ für Halogen, Y für Sauerstoff und $R^2$ für einen durch Halogenalkyl, Alkylmercapto, Alkylsulfonyl, Halogenalkylsulfinyl oder Halogenalkylsulfonyl mit jeweils bis zu 3 Kohlenstoffatomen einfach substituierten Phenylrest stehen.

4. Herbizid, enthaltend inerte Zusatzstoffe und ein 4H-3,1-Benzoxazinderivat der Formel I gemäss Anspruch 1.

5. Verfahren zur Herstellung von 4H-3,1-Benzoxazinderivaten der Formel I gemäss Anspruch 1, dadurch gekennzeichnet, dass man eine gegebenenfalls substituierte Anthranilsäure der Formel II

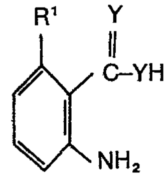

(II),

in der $R^1$ und Y die im Anspruch 1 genannten Bedeutungen haben, mit mindestens dem einfachen molaren Überschuss eines Carbonsäurehalogenids der Formel III

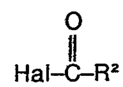

(III),

in der $R^2$ die im Anspruch 1 genannten Bedeutungen hat und Hal für Halogen steht, in einem aromatischen tertiären Amin als Lösungsmittel bei einer Temperatur im Bereich zwischen 10 und 60 °C umsetzt.

6. Verfahren zur Herstellung von 4H-3,1-Benzoxazinderivaten der Formel I gemäss Anspruch 1, dadurch gekennzeichnet, dass man eine gegebenenfalls substituierte Anthranilsäure der Formel II

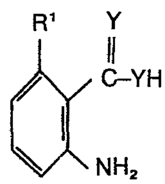

(II),

in der $R^1$ und Y die im Anspruch 1 genannten Bedeutungen haben, oder ein Alkalimetall- oder Erdalkalimetallsalz dieser Anthranilsäure mit ungefähr stöchiometrischen Mengen eines Carbonsäurehalogenids der Formel III

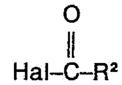

(III),

in der $R^2$ die im Anspruch 1 genannten Bedeutungen hat und Hal für Halogen steht, in einem inerten organischen Lösungsmittel oder in Wasser und gegebenenfalls in Anwesenheit eines Säureakzeptors bei einer Temperatur im Bereich von 0 bis 60 °C zu einem Carbonamid der Formel IV

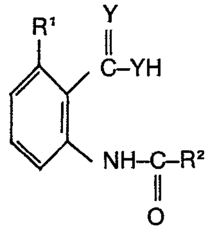

(IV),

in der $R^1$, $R^2$ und Y die im Anspruch 1 genannten Bedeutungen haben, umsetzt und dieses dann in Gegenwart eines wasserentziehenden Mittels bei einer Temperatur im Bereich zwischen 30 und 150 °C cyclisiert.

**Claims for the Contracting states:** BE, CH, DE, FR, GB, IT, LI, NL, SE

1. A 4H-3,1-benzoxazine derivative of the formula I

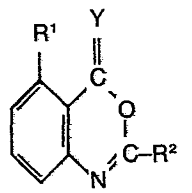

(I),

where

Y is oxygen or sulfur, and

$R^1$ is hydrogen if

$R^2$ is phenyl which is monosubstituted or disubstituted by haloalkenyl, haloalkenyloxy or haloalkenylmercapto, each of up to 3 carbon atoms, the substituents in the case of disubstitution being identical or different, or is phenyl which is monosubstituted by haloalkyl of up to 3 carbon atoms, with the exception of trifluormethyl or,

$R^1$ is halogen if

$R^2$ is phenyl which is monosubstituted by alkylmercapto, alkylsulfonyl, haloalkylsulfinyl or haloalkylsulfonyl, each of up to 3 carbon atoms, or is phenyl which is monosubstituted or disubstituted by haloalkyl, haloalkenyl, haloalkenyloxy or haloalkenylmercapto, each of up to 3 carbon atoms, the substituents in the case of disubstitution being identical or different.

2. A 4H-3,1-benzoxazine derivative of the formula I as claimed in claim 1, wherein $R^1$ is hydrogen, Y is oxygen and $R^2$ is phenyl monosubstituted by haloalkyl or haloalkenyl, each of up to 3 carbon atoms, with the exception of trifluoromethyl.

3. A 4H-3,1-benzoxazine derivative of the formula I as claimed in claim 1, wherein $R^1$ is halogen, Y is oxygen and $R^2$ is phenyl monosubstituted by haloalkyl, alkylmercapto, alkylsulfonyl, haloalkylsulfinyl or haloalkylsulfonyl, each of up to 3 carbon atoms.

4. A herbicide containing inert additives and a 4H-3,1-benzoxazine derivative of the formula I as claimed in claim 1.

5. A process for the control of unwanted plant growth, wherein a herbicide is used which contains at least one 4H-3,1-benzoxazine derivative of the formula I as claimed in claim 1.

6. A process for the preparation of a 4H-3,1-benzoxazine derivative of the formula I as claimed in claim 1, wherein an unsubstituted or substituted anthranilic acid of the formula II

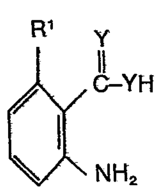

(II),

where $R^1$ an Y have the meanings given in claim 1, is reacted with at least a onefold molar excess of a carboxylic acid halide of the formula III

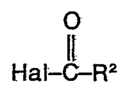

(III),

where $R^2$ has the meanings given in claim 1 and Hal is halogen, in an aromatic tertiary amine as the solvent, at from 10 to 60 °C.

7. A process for the preparation of a 4H-3,1-benzoxazine derivative of the formula I as claimed in claim 1, wherein an unsubstituted or substituted anthranilic acid of the formula II

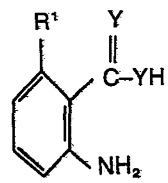

(II),

where $R^1$ and Y have the meanings given in claim 1, or an alkali metal salt or alkaline earth metal salt of this anthranilic acid, is reacted with the stoichiometric amount of a carboxylic acid halide of the formula III

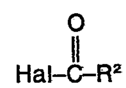

(III)

where $R^2$ has the meanings given in claim 1 and Hal is halogen, in an inert organic solvent or in water, in the presence or absence of an acid acceptor, at from 0 to 60 °C, to give a carboxamide of the formula IV

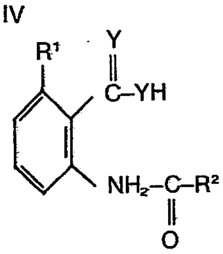

(IV),

where $R^1$, $R^2$ and Y have the meanings given in claim 1, and this compound is then cyclized in the presence of a dehydrating agent at from 30 to 150 °C.

**Claims for the Contracting state:** AT

1. A herbicide containing, as active ingredient, a 4H-3,1-benzoxazine derivative of the formula I

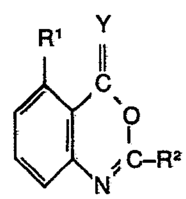

(I),

where

Y is oxygen or sulfur, and

$R^1$ is hydrogen if

$R^2$ is phenyl which is monosubstituted or disubstituted by haloalkenyl, haloalkenyloxy or haloalkenylmercapto, each of up to 3 carbon atoms, the

substituents in the case of disubstitution being identical or different, or is phenyl which is monosubstituted by haloalkyl of up to 3 carbon atoms, with the exception of trifluoromethyl or,

$R^1$ is halogen if

$R^2$ is phenyl which is monosubstituted by alkylmercapto, alkylsulfonyl, haloalkylsulfinyl or haloalkylsulfonyl, each of up to 3 carbon atoms, or is phenyl which is monosubstituted or disubstituted by haloalkyl, haloalkenyl, haloalkenyloxy or haloalkenylmercapto, each of up to 3 carbon atoms, the substituents in the case of disubstitution being identical or different.

2. A herbicide containing a 4H-3,1-benzoxazine derivative of the formula I as defined in claim 1, wherein $R^1$ is hydrogen, Y is oxygen and $R^2$ is phenyl monosubstituted by haloalkyl or haloalkenyl, each of up to 3 carbon atoms, with the exception of trifluoromethyl.

3. A herbicide containing a 4H-3,1-benzoxazine derivative of the formula I as defined in claim 1, wherein $R^1$ is halogen, Y is oxygen and $R^2$ is phenyl monosubstituted by haloalkyl, alkylmercapto, alkylsulfonyl, haloalkylsulfinyl or haloalkylsulfonyl, each of up to 3 carbon atoms.

4. A herbicide containing inert additives and a 4H-3,1-benzoxazine derivative of the formula I as defined in claim 1.

5. A process for the preparation of a 4H-3,1-benzoxazine derivative of the formula I as defined in claim 1, wherein an unsubstituted or substituted anthranilic acid of the formula II

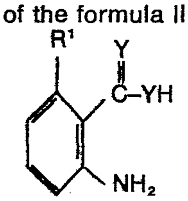 (II),

where $R^1$ and Y have the meanings given in claim 1, is reacted with at least a onefold molar excess of a carboxylic acid halide of the formula III

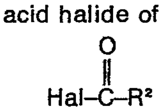 (III),

where $R^2$ has the meanings given in claim 1 and Hal is halogen, in an aromatic tertiary amine as the solvent, at from 10 to 60 °C.

6. A process for the preparation of a 4H-3,1-benzoxazine derivative of the formula I as claimed in claim 1, wherein an unsubstituted or substituted anthranilic acid of the formula II

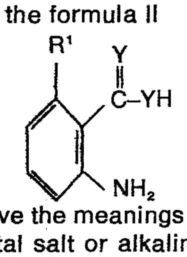 (II),

where $R^1$ and Y have the meanings given in claim 1, or an alkali metal salt or alkaline earth metal salt of this anthranilic acid, is reacted with about the stoichiometric amount of a carboxylic acid halide of the formula III

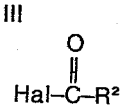 (III),

where $R^2$ has the meanings given in claim 1 and Hal is halogen, in an inert organic solvent or in water, in the presence or absence of an acid acceptor, at from 0 to 60 °C, to give a carboxamide of the formula IV

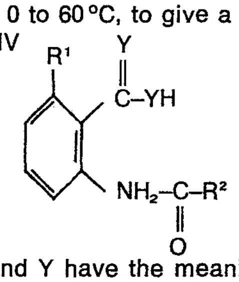 (IV),

where $R^1$, $R^2$ and Y have the meanings given in claim 1, and this compound is then cyclized in the presence of a dehydrating agent at from 30 to 150 °C.

**Revendications pour les Etats contractants:** BE, CH, DE, FR, GB, IT, LI, NL, SE

1. Dérivés de la 4H-3,1-benzoxazine de la formule I

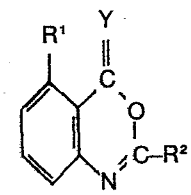 (I),

dans laquelle

Y désigne de l'oxygène ou du soufre et

$R^1$ de l'hydrogène,

si $R^2$ représente un groupe phényle mono- ou di-substitué par des radicaux halo-alcényle, halo-alcényl-oxy ou halo-alcényl-mercapto avec jusqu'à trois atomes de carbone identiques ou différents ou un groupe phényle mono-substitué par un radical halo-alkyle avec jusqu'à trois atomes de carbone, à l'exception du radical trifluorométhyle, ou

$R^1$ désigne un halogène,

si $R^2$ représente un groupe phényle mono-substitué par un radical alkyl-mercapto, alkyl-sulfonyle, halo-alkyl-sulfinyle ou halo-alkyl-sulfonyle avec jusqu'à trois atomes de carbone ou un groupe phényle mono- ou di-substitué par des radicaux halo-alkyle, halo-alcényle, halo-alcényl-oxy ou halo-alcényl-mercapto avec jusqu'à trois atomes de carbone identiques ou différents.

2. Dérivés de la 4H-3,1-benzoxazine de la formule I selon la revendication 1, caractérisés en ce que $R^1$ désigne un hydrogène, Y un oxygène et $R^2$ un groupe phényle mono-substitué par un radical halo-alkyle ou halo-alcényle avec jusqu'à trois atomes de carbone, à l'exception du radical trifluoro-méthyle.

3. Dérivés de la 4H-3,1-benzoxazine de la formule I selon la revendication 1, caractérisés en ce que $R^1$ désigne un halogène, Y un oxygène et $R^2$ un groupe phényle mono-substitué par un radical halo-alkyle, alkyl-mercapto, alkyl-sulfonyle, halo-alkyl-sulfinyle ou halo-alkyl-sulfonyle avec jusqu'à trois atomes de carbone.

4. Herbicide, contenant un dérivé de la 4H-3,1-benzoxazine de la formule I selon la revendication 1 et des additifs inertes.

5. Procédé de lutte contre la croissance de plantes indésirables, caractérisé en ce que l'on emploie un herbicide contenant au moins un dérivé de la 4H-3,1-benzoxazine de la formule I selon la revendication 1.

6. Procédé de préparation de dérivés de la 4H-3,1-benzoxazine de la formule I selon la revendication 1, caractérisé en ce que l'on fait réagir un acide anthranilique éventuellement substitué de la formule II

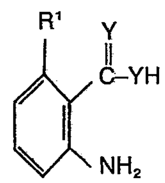

(II),

dans laquelle $R^1$ et Y possèdent les significations définies dans la revendication 1, dans une amine aromatique tertiaire comme solvant, à une température dans la gamme de 10 à 60 °C, avec au moins un excès molaire simple d'un halogénure d'acide carbonique de la formule III

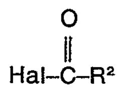

(III),

dans laquelle $R^2$ possède les significations définies dans la revendication 1 et Hal désigne un atome d'halogène.

7. Procédé de préparation de dérivés de la 4H-3,1-benzoxazine de la formule I selon la revendication 1, caractérisé en ce que l'on transforme un acide anthranilique éventuellement substitué de la formule II

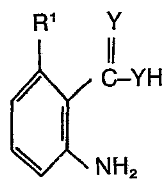

(II),

dans laquelle $R^1$ et Y possèdent les significations définies dans la revendication 1, ou un sel de métal alcalin ou de métal alcalino-terreux d'un tel acid anthranilique, par réaction avec la proportion stoechiométrique d'un halogénure d'acide carbonique de la formule III

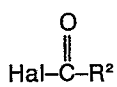

(III),

dans laquelle $R^2$ possède les significations définies dans la revendication 1 et Hal désigne un atome d'halogène, à une température dans la gamme de 0 à 60 °C dans un solvant organique inerte ou dans de l'eau, éventuellement en présence d'un fixateur d'acide, en un carbamide de la formule IV

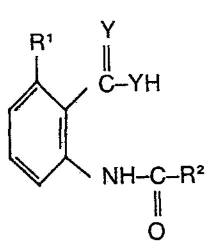

(IV),

dans laquelle $R^1$, $R^2$ et Y possèdent les significations définies dans la revendication 1, lequel est ensuite cyclisé à une température dans la gamme de 30 à 150 °C en présence d'un agent déshydratant.

**Revendications pour l'Etat contractant:** AT

1. Herbicide, contenant comme principe actif un dérivé de la 4H-3,1-benzoxazine de la formule I

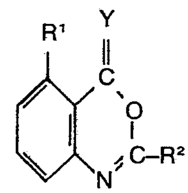

(I),

dans laquelle

Y désigne de l'oxygène ou du soufre et

$R^1$ de l'hydrogène,

si $R^2$ représente un groupe phényle mono- ou di-substitué par des radicaux halo-alcényle, halo-alcényl-oxy ou halo-alcényl-mercapto avec jusqu'à trois atomes de carbone identiques ou différents ou un groupe phényle mono-substitué par un radical halo-alkyle avec jusqu'à trois atomes de carbone, à exception du radical trifluoro-méthyle, ou

$R^1$ désigne un halogène,

si $R^2$ représente un groupe phényle mono-substitué par un radical alkyl-mercapto, alkyl-sulfonyle, halo-alkyl-sulfinyle ou halo-alkyl-sulfonyle avec jusqu'à trois atomes de carbone ou un groupe phényle mono- ou di-substitué par des radicaux halo-alkyle, halo-alcényle, halo-alcényl-oxy ou halo-alcényl-mercapto avec jusqu'à trois atomes de carbone identiques ou différents.

2. Herbicide, contenant un dérivé de la 4H-3,1-benzoxazine de la formule I selon la revendication 1, caractérisé en ce que $R^1$ désigne un hydrogène, Y un oxygène et $R^2$ un groupe phényle mono-substitué par un radical halo-alkyle ou halo-alcényle avec jusqu'à trois atomes de carbone, à l'exception du radical trifluoro-méthyle.

3. Herbicide, contenant un dérivé de la 4H-3,1-benzoxazine de la formule I selon la revendication 1, caractérisé en ce que $R^1$ désigne un halogène, Y un oxygène et $R^2$ un groupe phényle mono-substitué par un radical halo-alkyle, alkyl-mercapto, alkyl-sulfonyle, halo-alkyl-sulfinyle ou halo-alkyl-sulfonyle avec jusqu'à trois atomes de carbone.

4. Herbicide, contenant un dérivé de la 4H-3,1-benzoxazine de la formule I selon la revendication 1, ainsi que des additifs inertes.

5. Procédé de préparation de dérivés de la 4H-3,1-benzoxazine de la formule I selon la revendication 1, caractérisé en ce que l'on fait réagir un acid anthranilique éventuellement substitué de la formule II

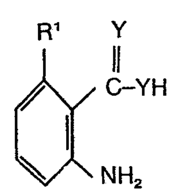

(II),

dans laquelle R¹ et Y possèdent les significations définies dans la revendication 1, dans une amine aromatique tertiaire comme solvant, à une température dans la gamme de 10 à 60 °C, avec au moins un excès molaire simple d'un halogénure d'acide carbonique de la formule III

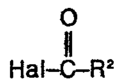

(III),

dans laquelle R² possède les significations définies dans la revendication 1 et Hal désigne un atome d'halogène.

6. Procédé de préparation de dérivés de la 4H-3,1-benzoxazine de la formule I selon la revendication 1, caractérisé en ce que l'on transforme un acid anthranilique éventuellement substitué de la formule II

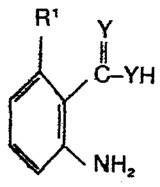

(II),

dans laquelle R¹ et Y possèdent les significations définies dans la revendication 1, ou un sel de métal alcalin ou de métal alcalino-terreux d'un tel acide anthranilique, par réaction avec la proportion stoechiométrique d'un halogénure d'acide carbonique de la formule III

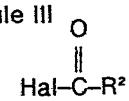

(III),

dans laquelle R² possède les significations définies dans la revendication 1 et Hal désigne un atome d'halogène, à une température dans la gamme de 0 à 60 °C dans un solvant organique inerte ou dans de l'eau, éventuellement en présence d'un fixateur d'acide, en un carbamide de la formule IV

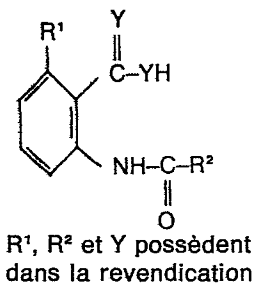

(IV),

dans laquelle R¹, R² et Y possèdent les significations définies dans la revendication 1, lequel est ensuite cyclisé à une température dans la gamme de 30 à 150 °C en présence d'un agent déshydratant.